(19) Europäisches Patentamt
European Patent Office
Office européen des brevets

(11) Publication number: **0 209 730 B1**

(12) **EUROPEAN PATENT SPECIFICATION**

(45) Date of publication of patent specification: **11.12.91** (51) Int. Cl.$^5$: **C07D 285/12**, C10M 135/36

(21) Application number: **86108385.5**

(22) Date of filing: **19.06.86**

The file contains technical information submitted after the application was filed and not included in this specification

(54) **Substituted 2,5-dimercapto-1,3,4-thiadiazoles and lubricating compositions containing same.**

(30) Priority: **26.07.85 US 759211**

(43) Date of publication of application:
**28.01.87 Bulletin 87/05**

(45) Publication of the grant of the patent:
**11.12.91 Bulletin 91/50**

(84) Designated Contracting States:
**BE CH DE FR GB IT LI LU NL SE**

(56) References cited:
**EP-A- 0 166 696**
**JP-A-53 037 418**
**US-A- 3 980 573**
**US-A- 4 306 988**
**US-A- 4 432 847**

(73) Proprietor: **R.T. VANDERBILT COMPANY, INC.**
**30 Winfield Street**
**Norwalk Connecticut 06855(US)**

(72) Inventor: **Karol, Thomas J.**
**33 Harbor View Avenue**
**Nowalk Connecticut 06855(US)**

(74) Representative: **Patentanwaltsbüro Cohausz & Florack**
**Schumannstrasse 97**
**W-4000 Düsseldorf 1(DE)**

EP 0 209 730 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

The present invention concerns novel derivatives of thiadiazole compounds and their use as functional additives for oil-based and water-based lubricating compositions. More particularly the new thiadiazoles are derived from 2,5-dimercapto-1,3,4-thiadiazole and epoxy compounds.

Additives known as antiwear agents are employed to increase the load-carrying capacity of lubricants. The antiwear agents promote the formation of a surface film and thereby prevent wear of the contacting surfaces.

During the course of use, lubricants are susceptible to deterioration due to oxidation. The oxidative process leads to the loss of lubricating properties and inadequate protection of the device to be lubricated. Antioxidants are added to inhibit the oxidative process. Thus, it is desirable that antiwear agents possess antioxidant properties.

Prior art has disclosed certain thiadiazole type compounds and their use as lubricating additives. However, due to stricter environmental controls, there is a need to develop new and effective ashless-type additives, preferably possessing multifunctional properties.

No art is known that teaches or suggests the present compounds or their use as multifunctional lubricating additives.

SUMMARY OF THE INVENTION

In accordance with the invention, there are provided novel reaction products of 2,5-dimercapto-1,3,4-thiadiazole and a substituted epoxy compound. The reaction products may be characterized by the structural formula

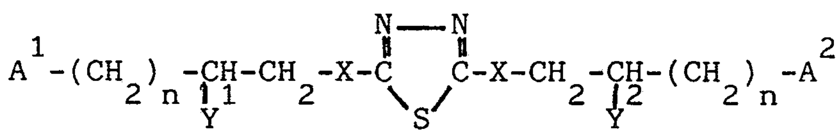

(I)

wherein
X represents -S-,

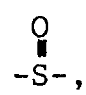

and

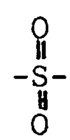

groups;
$Y^1$ and $Y^2$ represent -OH,

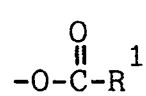

and

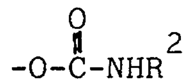

groups;

A¹ and A² represent an alkyl or alkoxy group with 1 to 100 carbon atoms;
R¹ and R³ represent an alkyl group with 1 to 100 carbon atoms; and
n is 1.

Another aspect of the invention concerns oil-based and water-based compositions containing the novel reaction products in an amount sufficient to impart antiwear and antioxidant properties.

PRIOR ART

US-A-3 980 573 and EP-A-0 166 696 disclose compounds presenting a close structural similarity and exhibiting an utility as multifunctional additives for oil-based lubricants.

DESCRIPTION OF SPECIFIC EMBODIMENTS

The reaction products of the invention may be prepared by reacting 2,5-dimercapto-1,3,4-thiadiazole with two molar equivalents of an epoxy compound to form the corresponding alcohol derivative. Further novel reaction products may be formed from the alcohol derivative by reacting with an alcohol reactive functional group. Thus, esters may be formed by esterification with an organic acid or an equivalent thereof as for example organic acid halide or organic acid anhydride. Other products may be formed by reacting the alcohol derivative with an isocyanate compound. All of the hereinabove described products may be oxidized by known methods to form the corresponding sulfonyl and sulfinyl derivatives.

The general reaction scheme is illustrated by the following equation wherein 2,5-dimercapto-1,3,4-thiadiazole is reacted with 2 moles of propylene oxide, as simple epoxide with A1 or A2 being hydrogen (not covered) to form the corresponding alcohol and then esterified with acetic anhydride.

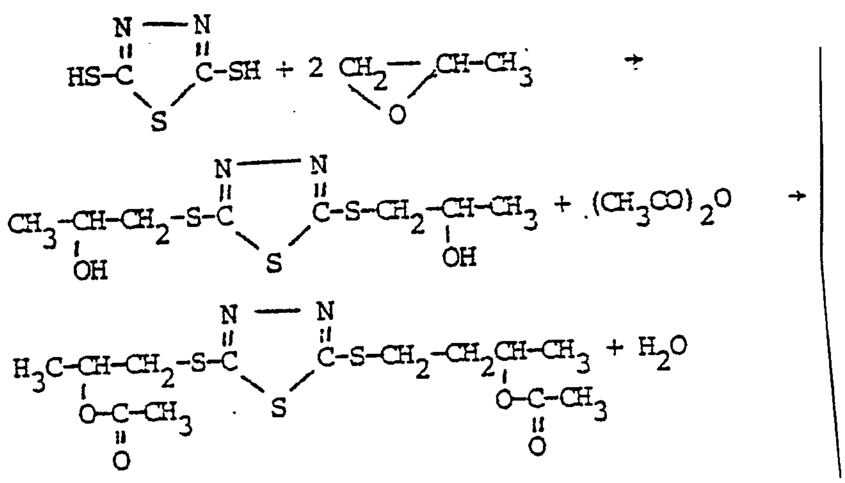

Preferably, the reaction with epoxides may be conducted in the presence of an inert solvent such as alcohols, toluene and benzene and a reaction promoter as for example tertiary amines. The reaction temperature will depend upon the specific reactants and solvent media employed. Typically reaction temperatures range from about 80 to 140°C.

The reaction illustrated by the equation is the preferred method. Other methods of synthesis may be used.

The groups (A¹ and A²) and (R¹ and R²) in formula (I) represent groups with 1 to 100 carbon atoms and a straight or branched chain. These include, among others, methyl, ethyl, n-propyl, iso-propyl, n-butyl, iso-butyl, sec-butyl, pentyl, octyl, dodecyl, octadecyl, polymeric alkyl, 2-ethoxyethyl, dodecyloxymethyl and butoxymethyl.

The alkyl groups (A¹) and (A²) may bethe same or different. Similarly the alkyl groups (R¹) and (R²) may be the same or different.

The thiadiazole derivatives of the invention are useful as lubricating additives. The compounds possess

multifunctional properties with respect to antiwear and oxidation inhibition. Although no particular theory is hereby relied on, the mercaptothiadiazoles are expected to be oxidized in performing the antioxidant role. These oxidized analogs still retain antiwear properties. Thus, the compounds are truly bifunctional.

The thiadiazoles may be incorporated into lubricating compositions. Preferred are thiadiazole reaction products of formula (I) wherein the radicals contain from 1 to 75 carbon atoms and preferably from 1 to 50 carbon atoms.

The lubricating compositions contemplated herein include lubricating oils and lubricating greases containing a major amount of petroleum hydrocabon and synthetic sources. The hydrocarbon base oil may be selected from naphthenic, aromatic, and paraffinic mineral oils. The synthetic oils may be selected from, among others, alkylene polymers, polysiloxanes, carboxylic acid esters and polyglycol ethers.

Another lubricating composition useful herein includes waterbased systems. Typically the aqueous systems comprise at least 40 percent of water and zero to less than 15 percent of base oil. Oil-soluble additives are incorporated in the system with the aid of solubilizer/stabilizer systems. The water based systems are useful not only as lubricants, but also as functional fluids such as cutting oils, hydraulic fluids, and transmission fluids.

The amount of the thiadiazole additive required to be effective for imparting antiwear and antioxidant characteristics in lubricating compositions may range from about 0.01 to 10 percent of the lubricating composition. The preferred range is about 0.1 to 5.0 percent of the additive of the total lubricating composition.

The lubricating compositions may contain the necessary ingredients to prepare the composition as for example dispersing agents, emulsifiers and viscosity improvers. Greases may be prepared by addition of thickeners as for example salts and complexes of fatty acids, polyurea compounds, clays and quaternary ammonium bentonite. Depending on the intended use of the lubricant, other functional additives may be added to enhance a particular property of the lubricant. The lubricating compositions may further contain known antioxidants, extreme pressure agents, metal passivators, rust inhibitors and other antiwear agents.

The following examples are given for the purpose of further illustrating the invention. All percentages and parts are based on weight unless otherwise indicated.

EXAMPLE 1

2,5-Bis(3-dodecyloxy-2-hydroxypropylthio-1,3,4-thiadiazole

2,5-Dimercapto-1,3,4-thiadiazole(79.9 grams, 0.53 moles) was charged into a reaction flask followed by isopropanol solvent, 200 ml. The epoxide, dodecyl glycidyl ether(272.4 grams, 1.06 moles) was added slowly to the reactor with stirring. The reaction mixture was heated at about 80 °C for 0.5 hours. At this time the solvent was stripped by using a rotary evaporator at approximately 20 mm Hg and 100 °C. The product was a low melting solid characterized by the infrared absorption bands at 3400, 2900, 1460, 1380, 1120, and 1050 $cm^{-1}$.

EXAMPLE 2

2,5-Bis(3-octyloxy-2-hydroxypropylthio-1,3,4-thiadiazole

The product was prepared substantially in accordance with the procedure described in Example 1 except that the epoxide used was octyl glycidyl ether. The product was characterized by the infrared absorption bands at 3400, 2900, 1460, 1380, 1260, 1120, and 1050 $cm^{-1}$.

EXAMPLE 3

2,5-Bis(3-butyloxy-2-acetoxypropylthio-1,3,4-thiadiazole

2,5-Dimercapto-1,3,4-thiadiazole (50.2 grams) andacetic anhydride (85 grams) were charged into a reaction flask. The epoxide, butyl glycidyl ether, was slowly added with warming to initiate the reaction. The reaction mixture was stirred at approximetely 80°C for 0.5 hours. Triethylamine (0.5 ml) was added and themixture was heated to 130°C for 0.5 hours. At this time the solvent was stripped by using a rotary evaporator at approximetely 20 mm Hg and 100°C.The product was characterized by the infrared absorption bands at 3450, 2900 1750, 1695, 1460, 1370, 1230, 1120, 1035, and 735 $cm^{-1}$.

EXAMPLE 4

2,5-Bis-(2-hydroxyhexadecylthio)-1,3,4-thiadiazole

2,5-Dimercapto-1,3,4-thiadiazole (50.03 grams) and 1,2-epoxyhexadecane (163 grams) were charged to the reaction flask with cooling to maintain the temperature below 100°C. The reaction mixture was stirred at 85 to 100°C for 0.5 hours. The reaction yielded a product which solidified below 85°C and was characterized by the infrared absorption bands at 3405, 2900, 1465, 1390, 1100, and 720 $cm^{-1}$.

EXAMPLE 5

2-(3-Dodecyloxy-2-hydroxypropylthio)-5-(3-butyloxy-2-hydroxypropylthio)-1,3,4-thiadiazole

2,5-Dimercapto-1,3,4-thiadiazole (51.64 grams, 0.34 moles) and isopropanol (150 ml) were added to the reaction flask. Dodecyl glycidyl ether (87.35 grams, 0.34 moles) was slowly added with cooling to maintain the temperature below 25°C. Then sodium hydroxide (0.05 grams) was added fllowed by butyl glycidyl ether (44.91 grams, 0.34 moles). The reaction mixture was refluxed for 15 minutes and the solvent was stripped off by using a rotary evaporator. The product was characterized by infrared absorption bands at 3200, 2900, 1460, 1385, 1260, 1120,1050, and 710 $cm^{-1}$.

EXAMPLE 6

2,5-Bis-(3-butyloxy-2-hydroxypropylthio)-1,3,4-thiadiazole

The product was prepared substantially in accordance with the procedure described in Example 1 except that the epoxide used was butyl glycidyl ether. The product was a light yellow liquid which was characterized by infrared absorption bands at 3400, 1460, 1385, 1120, 1040,and 740 $cm^{-1}$.

EXAMPLE 7

2,5-Bis-(2-acetoxyhexadecylthio)-1,3,4-thiadiazole

The product of Example 4 (1.5 grams), acetic anhydride (2 grams), toluene (5 ml), and triethylamine (0,1 grams) were charged into a reaction vessel and rfluxed for one hour. The solvent was stripped off by using a rotary evaporator at about 20 mm Hg and 100°C. The product was characterized by infrared absorption bands at 2900, 1750, 1460, 1370, 1230, and 1035 $cm^{-1}$.

EXAMPLE 8

2,5-Bis(3-dodecyloxy-2-hydroxypropylsulfonyl)-1,3,4-thiadiazole

The product of Example 1 (51.10 grams, 0.77 moles) and n-propanol (250 ml) were charged into a reaction vessel. A solution of 70% hydrogen peroxide (19.1 grams, 0.39 moles) was slowly added and the reaction mixture was refluxed for 0.5 hours. The product was extracted with about 150 ml of hexane andwashed with five 300 ml portions of water. The extracted layer was dried over magnesium sulfate, filtered, and stripped of the solvent as in Example 1. The produkt was characterized by infrared absorption bands at 3400, 2900, 1750, 1710, 1465, 1380, 1250, and 1120 $cm^{-1}$.

EXAMPLE 9

2,5-Bis(2-(butylcarbamoyl)-butylthio)-1,3,4-thiadiazole

2,5-Dimercapto-1,3,4-thiadiazole (30.92 grams, 0.206 moles) and hexane (30 ml) were charged into the reaction vessel and warmed. 1,2-Epoxybutane (30.2 grams, 0.419 moles) was slowly added with stirring and the mixture was refluxed for 0.5 hours with vigorous stirring. Triethylamine (0.5 ml) and butyl isocyanate (41.5 grams, 0.419 moles) were added and the mixture was refluxed for one hour. The solvent was stripped off by using a rotary evaporator and the solution was filtered. The product was characterized by infrared bands at 3360, 2950, 1700, 1460, 1385, 1250, 1115, 1040, and 970 $cm^{-1}$.

EXAMPLE 10

2,5-Bis(2-acetoxybutylthio)-1,3,4-thiadiazole

The product was prepared substantially in accordance with Example 3 except that the epoxide was 1,2-epoxybutane. The product was characterized by the infrared bands at 2950, 1740,1690, 1370, 1230, and 1095 $cm^{-1}$.

EXAMPLE 11

2,5-Bis(2-hydroxy-3-oleoyloxypropyl)-1,3,4-thiadiazole

The product was prepared substantially by the method described in Example 6 except that the epoxide was glycidyl oleate. The product was mixed with an equivalent amount of diluent mineral oil and filtered. The product was characterized by the infrared absorption bands at 3440, 2900, 1740, 1460, 1380, 1250, 1170, 1110, 1050, 960, and 720 $cm^{-1}$.

EXAMPLE 12

2,5-Bis(2-acetoxy-3-oleoyloxypropyl)-1,3,4-thiadiazole

The product of Example 11 was reacted with acetic anhydride according to the procedure described in Example 7. The product was characterized by the infrared absorption bands at 2900, 1750, 1460, 1370, 1230, 1160, 1050, 960, and 725 $cm^{-1}$.

EXAMPLE 13

The products of the invention were evaluated by the following tests:

1. Shell Four-Ball Wear Test

The test was conducted essentially according to the method described in ASTM D2266 procedure. Four highly polished steel balls 12.5 mm indiameter were placed in a test cup and submergedin the test sample. The test oil was SunvisTM21 manufactured by Sun Oil Company. The test was carried out at a rotation speed of 1800 rpm under a load of 20 kg at 54.5°Cand 40 kg at 93°C for 60 minutes. The diameter of wear scar produced by samples containing additives of the invention was measured and the data compiled in Table I. The data indicate that the present additives have excellent antiwear properties even when present in quantities less than 0.01 percent.

TABLE I

Four-Ball Wear Test

| Sample | | Percent | Scar Diameter, mm | |
|---|---|---|---|---|
| | | | 20 kg | 40 kg |
| 1 | None | — | 0.68 | 2.00 |
| 2 | 2,5-Bis(3-butoxy-2-acetoxypropylthio)-1,3,4-thiadiazole | 0.50 | 0.51 | 0.79 |
| 3 | 2,5-Bis(3-butoxy-2-acetoxypropylthio)-1,3,4-thiadiazole | 0.05 | — | 0.84 |
| 4 | 2,5-Bis(2-hydroxyhexadecylthio)-1,3,4-thiadiazole | 0.50 | 0.33 | 0.76 |
| 5 | 2,5-Bis(2-hydroxyhexadecylthio)-1,3,4-thiadiazole | 0.05 | — | 0.75 |
| 6 | 2,5-Bis(3-dodecyloxy-2-hydroxypropylthio)-1,3,4-thiadiazole | 0.50 | 0.49 | — |
| 7 | 2,5-Bis(3-dodecyloxy-2-hydroxypropylthio)-1,3,4-thiadiazole | 0.125 | — | 0.73 |
| 8 | 2,5-Bis(3-dodecyloxy-2-hydroxypropylthio)-1,3,4-thiadiazole | 0.0312 | — | 0.79 |
| 9 | 2,5-Bis(3-dodecyloxy-2-hydroxypropylsulfonyl)-1,3,4-thiadiazole | 0.50 | 0.57 | — |
| 10 | 2,5-Bis(3-dodecyloxy-2-hydroxypropylsulfonyl)-1,3,4-thiadiazole | 0.063 | — | 0.88 |
| 11 | 2,5-Bis(3-dodecyloxy-2-hydroxypropylsulfonyl)-1,3,4-thiadiazole | 0.0312 | — | 0.86 |
| 12 | 2,5-Bis(3-dodecyloxy-2-hydroxypropylsulfonyl)-1,3,4-thiadiazole | 0.0156 | — | 0.80 |
| 13 | 2,5-Bis(3-dodecyloxy-2-hydroxypropylsulfonyl)-1,3,4-thiadiazole | 0.0078 | — | 0.76 |

2. Timken Test

Lithium 12-hydroxystearate grease was formulated with 2,5-bis(3-dodecyloxy-2-hydroxypropylthio)-1,3,4-thiadiazole in the amounts given in Table II. The antiwear performance under extra pressure conditions was evaluated by the Timken Test according to ASTM D-2907 procedure. The data compiled in Table II indicate excellent performance by the additive of the invention.

TABLE II

Timken Test Data

| Sample | Additive Percent | Timken Pass Load, kg |
|---|---|---|
| 14 | None | 20 |
| 15 | 2.0 | 30 |
| 16 | 3.0 | 55 |

3. Thin Film Oxygen Uptake Test

The test was conducted essentially according to the method described by Chia-Soon Ku et. al., J. Am. Soc. Lubricating Eng., 40,2 75-83, 1984. The oxidation induction time of the lubricant was measured under conditions which simulate the high temperature oxidation processes in automotive engines by a modified rotary bomb oxidation test method ASTM D-2272. The test was conducted with 1.5 gram samples of SAE-30 Oil formulated with catalyst obtained from the National Bureau of Standards. 2,5-Bis(3-dodecyloxy-2-hydroxypropylthio)-1,3,4-thiadiazole was added to the package in the amounts indicated in Table III. The test was conducted at 160°C and initial oxygen pressure of 620.6 KPa(90 psi). A "pass" oil has a high induction time, while a "fail" oil has a low induction time. The additive of the invention has good antioxidant properties as shown by data compiled in Table III.

TABLE III

Thin Film Oxygen Uptake Test

| Sample | Additive Percent | Average Induction Time, Min. |
|---|---|---|
| 17 | None | 55 |
| 18 | 0.35 | 98 |
| 19 | 0.50 | 105 |

The above embodiments have shown various aspects of the present invention.

**Claims**

1. A 2,5-dimercapto-1,3,4-thiadiazole compound having the structural formula

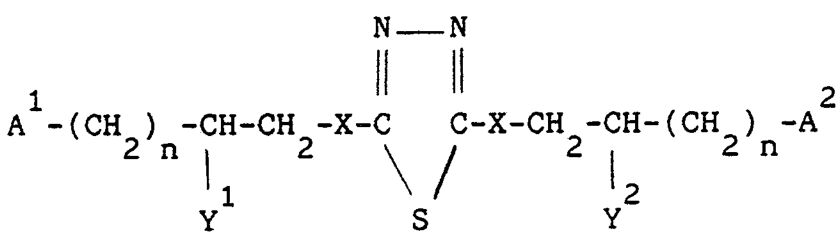

(I)

wherein X represents -S-,

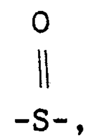

and

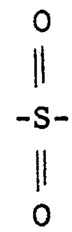

groups;

$Y^1$ and $Y^2$ represent -OH,

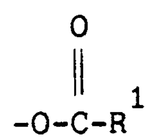

and

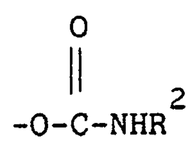

groups;

$A^1$ and $A^2$ represent an alkyl or alkoxy group with 1 to 100 carbon atoms,

$R^1$ and $R^2$ represent an alkyl group with 1 to 100 carbon atoms; and

n is 1.

2. The compound according to claim 1 which is 2,5-bis-(3-dodecyloxy-2-hydroxypropylthio)-1,3,4-thiadiazole.

3. The compound according to claim 1 which is 2,5-bis-(2-hydroxy-hexadecylthio)-1,3,4-thiadiazole.

4. The compound according to claim 1 which is 2,5-bis-(2-acetoxy-hexadecylthio)-1,3,4-thiadiazole.

5. A composition comprising a major amount of oil of lubricating viscosity wherein said oil is a petroleum hydrocarbon oil or a synthetic oil and from about 0.1 to 10 percent by weight of a product of any of claims 1 to 4.

## Revendications

1. Composé 2,5-dimercapto-1,3,4-thiadiazole de formule développée

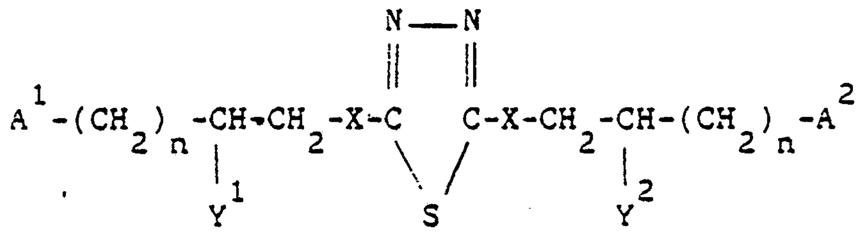 (I)

dans laquelle
X représente des groupes -S-,

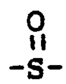

et

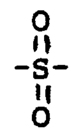;

$Y^1$ et $Y^2$ représentent des groupes -OH,

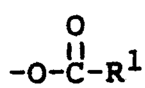

et

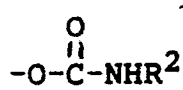;

$A^1$ et $A^2$ représentent un groupe alkyle ou alcoxy comportant 1 à 100 atomes de carbone;
$R^1$ et $R^2$ représentent un groupe alkyle comportant 1 à 100 atomes de carbone; et
n est égal à 1.

2. Composé selon la revendication 1, qui est le 2,5-bis(3-dodécyloxy-2-hydroxypropylthio)-1,3,4-thiadiazole.

3. Composé selon la revendication 1, qui est le 2,5-bis(2-hydroxy-hexadécylthio)-1,3,4-thiadiazole.

4. Composé selon la revendication 1, qui est le 2,5-bis(2-acétoxy-hexadécylthio)-1,3,4-thiadiazole.

5. Composition comprenant une quantité majeure d'huile de viscosité lubrifiante, dans laquelle ladite huile est une huile hydrocarbonée de pétrole ou une huile synthétique, et d'environ 0,1 à 10% en poids d'un produit selon l'une quelconque des revendications 1 à 4.

**Patentansprüche**

1. 2,5-Dimercapto-1,3,4,-thiadiazol der Formel

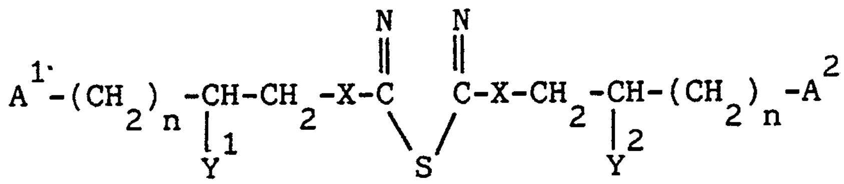 (I)

worin X -S-,

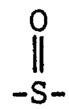

und

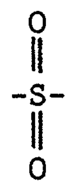

Gruppen darstellen,

$Y^1$ und $Y^2$ -OH,

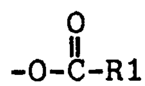

und

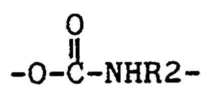

Gruppen dastellen,

| | |
|---|---|
| $A^1$ und $A^2$ | eine Alkyl- oder Alkoxy-Gruppe mit 1 bis 100 Kohlenstoffatomen darstellen, |
| $R^1$ und $R^2$ | eine Alkylgruppe mit 1 bis 100 Kohlenstoffatomen darstellen, und |
| n | 1 ist. |

**2.** 2,5-Bis-(3-dodecyloxy-2-hydroxypropylthio)-1,3,4-thiodiazol.

**3.** 2,5-Bis-(2-hydroxy-hexadecylthio)1,3,4-thiodiazol.

**4.** 2,5-Bis-(2-acetoxy-hexadecylthio)-1,3,4-thiodiazol.

**5.** Produkt, das zu einem größeren Teil aus einem Öl von Schmierviskosität besteht, worin dieses Öl ein Petroleumkohlenwasserstofföl oder ein synthetisches Öl ist und etwa 0,1 bis 10 Gew.-% einer Verbindung gemäß den Ansprüchen 1 bis 4 enthält.